# EUROPEAN PATENT APPLICATION

(11) **EP 2 559 484 A1**
(43) Date of publication of application: **20.02.2013**
(21) Application number: 11177920.3
(22) Date of filing: 18.08.2011
(51) Int. Cl.: B01J 31/24

(54) **Coordination complex system comprising a second bulding block without donor moiety**

(71) Applicant: Universiteit van Amsterdam, 1012 WX Amsterdam (NL)
(72) Inventor: Reek, Joost Nicolaas Hendrik, 3817 BK Amersfoort (NL); Dydio, Pawel Franciszek, 38-500 Sanok (PL)
(74) Representative: De Vries & Metman

(57) **Abstract**

The invention relates to a coordination complex system for chemically converting a substrate comprising a non-protein ligand having a first building block with at least one functional group and at least one donor moiety, which donor moiety is complexed to a metal selected from a transition metal and lanthanide or an organometallic precursor thereof, characterized in that the ligand further comprises at least one second building block not having a donor moiety and having at least one functional group that is complementary to the at least one functional group of the first building block, wherein the first building block and the second building block are non-covalently bonded through their functional groups. These coordination complex systems are used as a catalyst for chemical reactions such as hydroformylation, hydrogenation, transfer hydrogenation, hydrocyanation, polymerization, isomerization, carbonylation, cross-coupling, metathesis, CH-activation, allylic substitution, aldol condensation, and Michael addition, preferably to obtain chiral compounds.

## Description

The invention relates to a coordination complex system for chemically converting a substrate comprising a non-protein ligand having a first building block with at least one functional group and at least one donor moiety, which donor moiety is complexed to a metal selected from a transition metal and lanthanide or an organometallic precursor thereof, to the use of said coordination complex system as a catalyst, to a method of preparing said catalyst, and to a method of selecting an optimal second building block.

During the past decades combinatorial chemistry has evolved in an incredible manner and has been applied especially in pharmacy for drug discovery and optimization and for making homogeneous catalysts. The development of homogeneous catalysts using combinatorial techniques involves two distinct challenges: 1) devising strategies and methods for the preparation of large libraries of ligands and/or catalyst displaying high degrees of structural diversity, 2) developing high-throughput screening techniques for the reaction of interest. A lot of effort has been put in the development of new screening techniques for homogeneous catalyst libraries and various methods have proven successful. In the search for new transition metal catalysts the preparation of catalyst libraries has mainly been focused on variation of ligands, which are based on commercially available ligands or are prepared via conventional synthetic pathways and/or divergent methods using parallel synthesis. The synthesis of large libraries of new ligands indeed yields a major challenge and so far only a limited number of methodologies have been reported. Until now, synthesis of libraries of ligands is based on combinatorial organic synthesis followed by metal complexation. This approach utilizes advanced solid and solution phase combinatorial synthetic methodologies including parallel synthesis, split-pool techniques, encoding/deconvolution and polymer-supported reagents. Methods such as split pool techniques are much faster than traditional serial approaches and enable the preparation of relatively large numbers of compounds but often lack control over the purity and mixtures of compounds entering the assay screen. In contrast, the methods based on parallel or array syntheses yield intermediate sized libraries of pure compounds. Although these combinatorial techniques to construct ligand libraries have proven to be valuable, the preparation and evaluation of truly large numbers of potentially (enantio)-selective catalysts has just been started. The preparation of important multidentate ligands, like promising diphosphorus ligands, still faces synthetic challenges, especially when sophisticated chiral entities are required for asymmetric catalysis.

A substantial improvement to these techniques was disclosed in WO 2004/103559, wherein a flexible and versatile synthesis of tailor-made catalytic systems for metal-catalyzed reactions was proposed. According to the method described therein a coordination complex system comprising a ligand having at least two donor moieties was made. Each of these building blocks contains one or more functional groups and one or more donor moieties. The donor moieties are able to coordinate with a transition metal or lanthanide. This techniques enables the preparation of a number of coordination complex systems by combining building blocks to new ligands. However, this method appears to have serious drawbacks. Since both building blocks contain donor moieties, variation of building block changes both the selectivity and the activity of the coordination complex. As such, some changes may imply improvement of selectivity at the expense of the activity.

Thus the first building block has to be selected to optimize the activity of the catalyst. Having optimized the first building block the second building block has to be selected to optimize the selectivity of the catalyst. Due to the presence of a donor moiety on this second building block, by optimizing the selectivity the activity may become sub-optimal. Hence, after the second building block of the ligand has been found, the first building block should sometimes be optimized again.

Hence there is a need for a more convenient method wherein the fast production of many coordination complex systems (catalysts) is still secured, but wherein the optimization of the selectivity does not lead to decrease of the activity of the catalyst. The present invention provides a solution to this problem.

According to this invention a coordination complex system for chemically converting a substrate is obtained comprising a non-protein ligand having a first building block with at least one functional group and at least one donor moiety, which donor moiety is complexed to a metal selected from a transition metal and lanthanide or an organometallic precursor thereof, characterized in that the ligand further comprises at least one second building block not having a donor moiety and having at least one functional group that is complementary to the at least one functional group of the first building block, wherein the first building block and the second building block are non-covalently bonded through their functional groups.

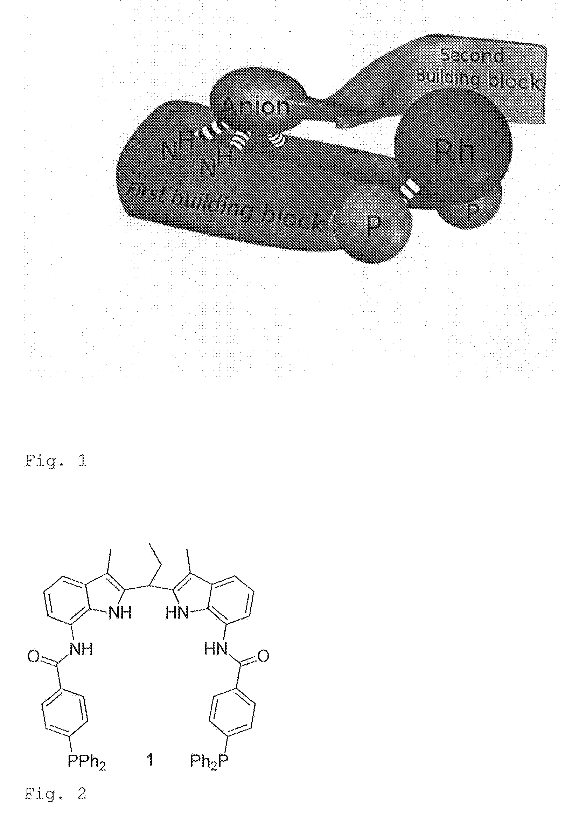

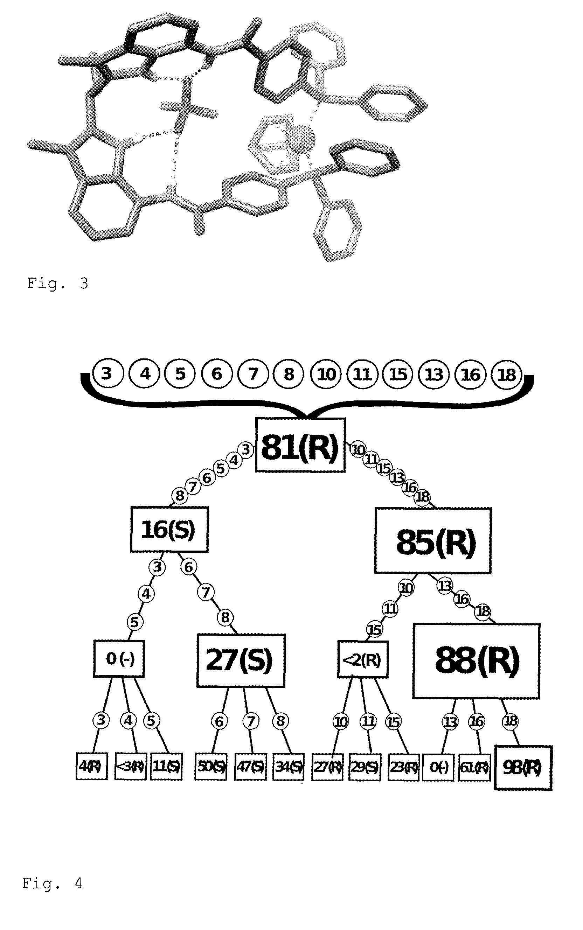

In Fig. 1 the coordination complex system of this invention is schematically represented. The first building block comprises two donor moieties ("P") that are complex with a metal ("Rh"). A second building block comprises a functional group ("anion") which forms a non-covalent bond with an NH functional group of the first building block. In Fig. 2 a specific example of a first building block is given wherein the donor moieties are two Ph₂P (diphenylphosphine) groups and the NH groups of the diamidodiindolylmethane moiety are the functional groups. Fig. 3 shows the coordination complex system of [Rh(1)(nbd)BF₄] (nbd=norbornadiene) of one of two independent complexes found in the solid state. Hydrogen atoms (except for NH) and CH₂Cl₂ solvent molecules have been omitted for clarity. The coordination complex of this invention may contain the metal as such or an organometallic precursor thereof.

Hence in comparison with WO 2004/103559, the second building block having a donor moiety has been replaced by a second building block not having a donor moiety, but still, as the second building block of WO 2004/103559, having a functional group that is complementary to the functional group of the first building block to form the ligand. It was found that the optimized activity by selection of the optimal first building block of WO 2004/103559) is not longer affected when optimizing the second building block. It is preferred to use the first and second building blocks in about stoichiometric amounts, as it was found that the activity and selectivity of the ligands so obtained are then the most optimal ones. The present method therefore provides a further improvement of the number of experiments that are necessary to find the best catalyst.

The coordination complex systems of this invention can be used in the same manner as previously described in WO 2004/103559, which methods are included by reference. Thus the coordination complex systems can, if one whishes, be attached to a solid support, and can be used in a variety of chemical reactions to a substrate, including hydroformylation, hydrogenation, transfer hydrogenation, hydrocyanation, polymerization, isomerization, carbonylation, cross-coupling, metathesis, CH-activation, allylic substitution, aldol condensation, and Michael addition reactions of the substrate.

The term "non-covalent" as used herein has the common meaning as used by the skilled person, i.e. any chemical or physical bonding that is not covalent. Non-covalent bonding, for example, includes bonding via ionic interactions, hydrogen bonding, and reversible metal-ligand interactions.

The term "first building blocks" means any chemical entity with the exception of proteins. Preferably, first building blocks have a molecular weight less than 5,000, more preferably less than 2,500.

Donor groups for transition metals and lanthanides are well known (see for instance: C.Michael Hogan. 2010. Heavy metal. Encyclopedia of Earth. National Council for Science and the Environment. eds E. Monosson & C. Cleveland, Washington DC) and include phosphine groups.

Organometallic precursors are well-known organometallic compounds that are usually commercially available and are commonly used as the source of a metal. The coordination complex of this invention may contain the organic moiety that originates from the precursor, or it may be free from that organic moiety. Usually the coordination complex (catalyst) contains such organic moiety at the start of the reaction, but during the reaction this moiety will usually be split off, thereby forming the catalyst without such organic moiety.

The term "second building block" means any non-protein chemical entity having a functional group (that is complementary to the functional group of the first building block). Second building blocks do not contain a donor moiety and thus cannot form a bond with a metal. Second building blocks are usually chemical compounds with relatively low molecular weight, such as less than 1000, preferably less than 500. The functional groups are selected to be complementary with the functional groups of the first building block, and optionally a further functional group is selected to be complementary with a functional group of the substrate. Hence, if for instance the first building block contains hydrogen bond-donor groups such as NH, the second building block should contain a hydrogen bond-accepting group, such as a carboxylate, a phosphate, a sulfonate, and the like. If the first building block has an acid group as functional group, the second building block may contain an amine group as the complementary functional group. For first building blocks having functional NH groups, suitable second building blocks are for instance natural aminoacids, because these may contain as chiral center which render these suitable for obtaining catalysts for chiral synthesis.

In an embodiment of the invention the ligand (or alternatively the first building block) may be immobilized onto an inorganic support, a polymeric organic support, or a hybrid support.

The present method is particularly suitable for creating a chiral environment. This can be done by using chiral second building blocks, such as natural chiral acids, for instance aminoacids, chiral first building blocks, or both chiral first building blocks and chiral second building blocks. Preferably the first building block is achiral and the second building block is chiral. In such systems the second building block controlled catalysis is a powerful method to obtain selective catalysts for the asymmetric hydrogenation, as selectivity between 50 (S) and 99 (R) ee (enantiomeric excess) can be achieved. The first and second building blocks preferably only contain covalent bonds, hence do not contain non-covalent bonds.

The coordination complex system preferably has a molar ratio between the first building block and the metal of between 0.2 and 100.

The invention further relates to methods of preparing a catalyst. A preferred method is the one wherein in a first step a non-protein first building block having at least one functional group and at least one donor moiety is selected from a library of first building blocks. In a second step a second building block not having a donor moiety and having at least one functional group that is complementary to the functional group of the first building block is selected from a library of second building blocks. The coordination complex system is then obtained by bringing in contact in any order the first building block, the second building block and a metal selected from a transition metal and lanthanide or an organometallic precursor thereof. Hence, the first building block and the second building block may first be brought together and then the metal or the organometallic precursor thereof is added, or the first building block is first complexed with the metal (or the organometallic precursor thereof) after which the second building block is added, or the second building block together with the metal (or organometallic precursor) are added to the first building block, or the metal (or organometallic precursor), first building block and second building block are added simultaneously. Thereby a non-covalent bond is formed between a functional group of the first building block and a functional group of the second building block (which is possible because the functional group of the second building block is complementary to the functional group of the first building block) and a complex is formed between at least one of the donor moieties of the first building block and the metal (or organometallic precursor).

According to another method a non-protein first building block having at least one functional group and one donor moiety is selected from a library of first building blocks. Then the first building block is brought in contact in any order with a plurality of second building blocks, none of which having a donor moiety and each having at least one functional group that is complementary to the functional group of the first building block, and with a metal selected from a transition metal and lanthanide or an organometallic precursor thereof, thereby forming a non-covalent bond between a functional group of the first building block and a functional group of at least one of the second building blocks, and by forming a complex between at least one of the donor moieties of the first building block and the metal (or organometallic precursor). Because the first building block will exclusively, or at least predominantly, form a non-covalent bond with the second building block having the greatest interaction, or if two or more second building block have a similar interaction only with these second building blocks, the method only renders one, or at least a minor number of complexes, despite that a plurality of second building blocks was used. It can also be possible that several coordination complexes are present in solution, but that the reaction with the substrate is dominated by the one complex based on the best second building block as this gives the fasted reaction.

Alternatively, not a plurality of second building blocks may be used, but a plurality of first building blocks. According to this alternative a second building block not having a donor moiety and having at least one functional group is selected from a library of second building blocks. The catalyst is then formed by bringing in contact in any order the selected second building block, a plurality of first building blocks, each having at least one functional group that is complementary to a functional group of the second building block and each having at least one donor moiety, and a metal selected from a transition metal and lanthanide or an organometallic precursor thereof. For the same reasons as explained herein above only one, or at least a limited number of catalysts are obtained by forming a non-covalent bond between a functional group of the second building block and a functional group of at least one of the first building blocks, and by forming a complex between at least one of the donor moieties of the first building block and the metal (or organometallic precursor).

According to another embodiment of the invention a method is provided to facilitate the search to an optimal second building block. For instance, if the best first building block is found in terms of obtaining the best catalyst for rendering the best activity for a certain chemical reaction, and a library of 100 second building blocks is available, then 100 catalysts can be made and tested in the chemical reaction for their selectivity. This is quite a burden, and this search can considerably be shortened from 100 experiments to only 6 or 7. If in general terms a library of n second building blocks is available, wherein n is an integer of at least 3, the method of selecting a second building block is as follows.

The library of n second building blocks is divided in a sub-library with m second building blocks and a sub-library with n-m second building blocks, wherein n and m are integers. The number m is preferably selected for efficiency reasons so that m=½n for even numbers n, and for uneven numbers n so that m=½(n+1).

The libraries with m and n-m second building blocks are used to make the catalyst by making the complex with the first building block and the metal, as explained herein above. This gives a catalyst library A and a catalyst library B, respectively. These libraries consist of only one, or at least very few (effective) catalysts and for the most part unbound second building blocks.

These libraries are used as such to perform the catalytic reaction with the substrate that is under investigation. One of these catalyst libraries will give the best results (in terms of activity, selectivity, side-product formation, and the like). The best catalyst library was obtained by the best sub-library of second building blocks and this sub-library is selected and the other is discarded. The best sub-library is again divided in two parts (p and (m-p) or p and (n-m-p), wherein p is the number of second building blocks in this sub-library, and wherein p and m-p (or (n-m-p)are preferably the same number, or for uneven numbers of second building blocks p is one more than m-p or (n-m-p). These two new sub-libraries of second building blocks are again used to make the catalyst and are used in the chemical reaction to investigate which one has the best performance. The best library is again selected, the other discarded. This new sub-library is again divided in two parts (q and p-q or q and (m-p-q) or q and (n-m-p-q)), in the same manner, converted into the catalyst and tested in the chemical reaction. This procedure is repeated until the sub-library of second building blocks contains one second building block. The test of two sub-libraries of one second building block provides the information on which of the n second building blocks is the optimal second building block.

### Experimental part

### General

All reactions were carried out under an argon atmosphere using standard Schlenk techniques. THF, pentane, hexane and diethyl ether were distilled from sodium benzophenone ketyl; CH₂Cl₂, isopropanol and methanol were distilled from CaH₂ and toluene was distilled from sodium under nitrogen. NMR spectra were measured on a Bruker AMX 400 (400.1 MHz, 100.6 MHz and 162.0 for ¹H, ¹³C and ³¹P respectively). ¹⁹F NMR spectra were recorded on a Varian Mercury 300 (¹⁹F: 282.4 MHz).

Elemental analyses were carried out on a Carlo Erba NCSO-analyzer. High resolution mass spectra were recorded on a JEOL JMS SX/SX102A four sector mass spectrometer; for FAB-MS 3-nitrobenzyl alcohol was used as matrix. ESI-MS measurements were recorded on a Shimadzu LCMS-2010A liquid chromatography mass spectrometer by direct injection of the sample to the ESI probe. CD₂Cl₂ and DIPEA were dried over molecular sieves (4Å) and degassed by 3 freeze-pump-thaw cycles.

Conversions and enantiomeric excess for the asymmetric hydrogenation of methyl-2-acetamidoacrylate and ethyl-2-acetamidoacrylate were determined by Gas Chromatography on an Interscience Focus GC Ultra (FID detector) with a Supelco β-dex 225 column (internal diameter 0.25 mm, 30 m column, film thickness 0.25 µm). Conversions and enantiomeric excess for the asymmetric hydrogenation of benzyl-2-acetamidoacrylate were determined by chiral HPLC (column Chiralcel OD-H, flow rate: 1 mL/min, eluent: hexane/isopropanol (90/10), detection at 254 nm, 40 °C). All reagents were purchased from commercial suppliers and used without further purification, with the exception of the first building block 1, the anion receptor 20, 1-ethyl-2-acetamidoacrylate 21, benzyl 2-acetamidoacrylate 22 and methyl N-methyl-2-acetamidoacrylate 24, which were synthesized according to published procedures (P. Dydio, W.I. Dzik, M. Lutz, B. de Bruin, J.N.H. Reek, J.N.H.; Angew. Chem. Int. Ed., 2011, 396-400).

### General procedure for Rhodium-catalyzed hydrogenation reactions

The hydrogenation experiments were carried out in a stainless steel autoclave (150 mL or 250 mL) charged with an insert suitable for, respectively, 8 or 15 reaction vessels (including Teflon mini stirring bars) for conducting parallel reactions. In a typical experiment, the reaction vessels were charged with 1.0 µmole of [Rh(nbd)₂]BF₄, 1.1 µmole of first building block 1, 12.0 µmole of second building block, 9.0 µmole of N,N-diisopropylethylamine (DIPEA) and 0.1 mmole of substrate in 1.0 ml of CH₂Cl₂. Before starting the catalytic reactions, the charged autoclave was purged three times with 10 bar of dihydrogen and then pressurized at 10 bar H₂. The reaction mixtures were stirred at 25 °C for 16 hours. After catalysis the pressure was released and the conversion and enantiomeric purity were determined by chiral GC or chiral HPLC. pressure was released and the conversion and enantiomeric purity were determined by chiral GC or chiral HPLC.

### Synthesis of second building blocks

### (S)-N-acetyl-valine 10

The synthesis was realized according to the published procedure of N-acetyl-DL-alanine synthesis: (S)-valine (2 g, 17.07 mmole) and acetic acid anhydride (5.55 ml) were dissolved in methanol (10 ml), and the reaction mixture was heated at 70 °C for 6 h. After cooling down, all the volatiles were removed under vacuum. Ethyl acetate (20 ml) was added to the crude solid, followed by sonication on the ultrasonic bath for 1 h. Next, the product was filtered off, dissolved in DCM (10 ml) and precipitated by the slow addition of hexane, providing the powder which was isolated by filtration, yielding 1.84g (68%) of 10, according to ¹H-NMR (400MHz, DMSO-d₆) and ¹³C-NMR (100MHz, DMSO-d₆).

### (S)-N-formyl-valine 12

The synthesis was realized according to the published procedures of N-formyl-DL-alanine synthesis: (S)-valine (0.3 g, 2.5 mmole), formic acid (0.52 g, 11.3 mmole) and acid acetic anhydride (1 g, 9.8 mmole) were dissolved in acetic acid (10 mL), and the reaction was stirred for 4 h at room temperature. Afterwards, all the volatiles were removed under vacuum and the crude product was recrystallized from ethyl acetate, yielding 0.27 g (75%) of 12, according to ¹H-NMR (400MHz, DMSO-d₆) and ¹³C-NMR (100MHz, DMSO-d₆).

### (S)-N-trimethylacetyl-valine 11

The synthesis was realized according to the published procedures of N-acetamide of (S)-alanine synthesis: valine (0.5 g, 4.3 mmole) and trimethylacetic acid anhydride (2.4 mL, 11.5 mmole) were dissolved in methanol (10 mL), and the reaction mixture was heated to reflux overnight. Next, after cooling down, all the volatiles were removed under vacuum, the crude product was crystallized from hot hexane, and isolated by the filtration of cold solution (0°C), yielding 0.25 g (29%) of 11, according to ¹H-NMR (400 MHz, DMSO-d₆), ¹³C-NMR (100 MHz, DMSO-d₆), and elemental analysis.

### Synthesis of coordination complex systems

The cationic rhodium-first building block complex, [Rh(1)(nbd)]⁺, the precursor to the active hydrogenation catalyst, was easily obtained by mixing a CD₂Cl₂ solution of first building block 1 and [Rh(ndb)₂BF₄]. The NMR data of the complex showed that the two P donors are coordinated to the Rh center in a mutual cis orientation. This coordination geometry was further supported by the X-ray crystal structure of [Rh(1)(nbd)BF₄] (Figure 3). The crystal structure shows that in the solid state the BF₄ counter-anion is bound in the anion binding site. Binding studies carried out in solution demonstrate that the BF₄ bound in the pocket is quantitatively replaced by the second building blocks with carboxylate functional groups, which have a much higher affinity for the recognition site.

NMR experiments show that upon binding of simple chiral anions such as α-hydroxy acids, amino acids and their derivatives, in the pocket of free first building block 1 the P atoms as well as indole and amide NH's become diastereotopic, confirming chirogenesis - chirality transfer through supramolecular interactions. Importantly for catalytic purposes, this chirogenetic effect is also manifested when such chiral second building blocks are bound to the rhodium complex [Rh(1)(nbd)]⁺.

### Performance of complexes

Performance of the complexes were studied in the asymmetric hydrogenation of methyl 2-acetamidoacrylate (2), using α-hydroxy acids, amino acids as well as their N-protected derivatives, that are carbamates, amides, ureas and thioureas as second building blocks. Results are given in Table 1.

**Table 1. Asymmetric hydrogenation of 2 using [Rh(1)(nbd)]⁺ and first building block 1 (Fig. 2) in combination with various second building blocks^{a}.**

| | | | |
|---|---|---|---|
| 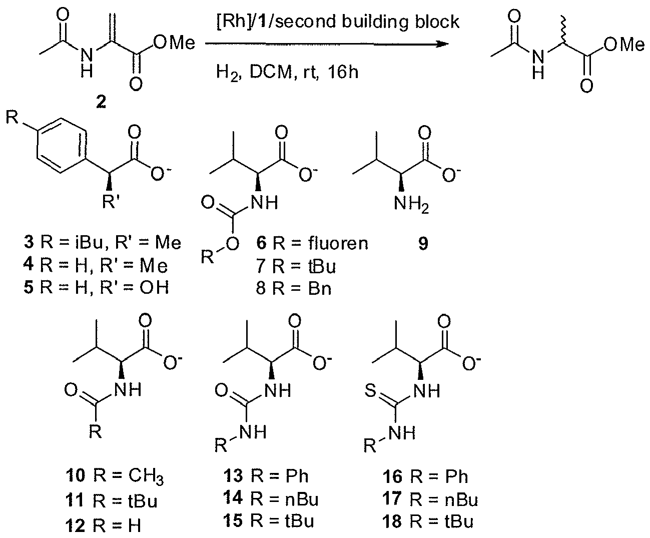 | | | |

| compound | Second building block | % conv. | % ee (config.) |
|---|---|---|---|
| 3 | R=iBu, R'=CH₃ | 100 | 4 (S) |
| 4 | R=H, R'=CH₃ | 100 | <3 (S) |
| 5 | R=H, R'=OH | 100 | 11 (S) |
| 6 | R=fluoren | 100 | 50 (S) |
| 7 | R=t-Bu | 100 | 47 (S) |
| 8 | R=Bz | 100 | 34 (S) |
| 9 | | 100 | 0 (-) |
| 10 | R=CH₃ | 100 | 27 (R) |
| 11 | R=t-Bu | 100 | 29 (S) |
| 12 | R=H | 100 | 0 (-) |
| 13 | R=Ph | 100 | 0 (-) |
| 14 | R=n-Bu | 100 | 0 (-) |
| 15 | R=t-Bu | 100 | 23 (R) |
| 16 | R=Ph | 3 | 61 (R) |
| 17 | R=n-Bu | 47 | 0 (-) |
| 18 | R=tBu | 3 | 98 (R) |
| 18^{b} | R=tBu | 18 | 99 (R) |
| 18^{c} | R=tBu | 100 | 98 (R) |

| | | | |
|---|---|---|---|
| Reactions were performed in dichloromethane (DCM), Rh/1/second building block/N,N-diisopropylethylamine (DIPEA)/2 =1:1.1:12:9:100; C(Rh)=0.001M, 10 bar H₂, at RT for 16 h, using [Rh(nbd)₂BF₄] as metal precursor; DIPEA was used as a base to deprotonate acidic second building block. Conversion and ee were determined by chiral GC analysis of the reaction mixture; ^{b} Rh/second building block/DIPEA = 1:6:2; ^{c} Rh/second building block/DIPEA = 1:3:2. | | | |

Under mild conditions (1% catalyst, 10 bar of H₂, 298 K, 16 h) complete conversion was obtained in most experiments (Table 1). The best results were found among the amino acids derivatives. The results show that the selectivity of the reaction is most sensitive to changes on the N-group of the second building block, whereas variation of the side group (that is by using derivatives of different amino acids) has much smaller influence. The highest selectivity was obtained when tert-butyl thiourea 18 was applied as second building block, which gave the product R with excellent enantioselectivity 98% ee.

Control experiments using triphenylphosphine (19) as the first building block in presence of the most effective second building blocks (carbamate 6 and thiourea 18), gave the product in a racemic form, indicating that the second building block needs the binding site to affect the metal complex (Table 2). In a separate control experiment a mixture of anion receptor 20, triphenylphospine (19) and the second building blocks (6 and 18) was used, and also in these reactions the product was formed in a racemic form. Hydrogenation of 2 in the presence of second building block 6 or 18, in the absence of any phosphorous ligand, gave the product again with no selectivity (ee = 0%). These control experiments demonstrate that the binding site must be an integrated part of the ligand, nearby the metal center.

**Table 2. Asymmetric hydrogenation of 2 and 24 - control experiments^{a}**

| | | | | |
|---|---|---|---|---|
| 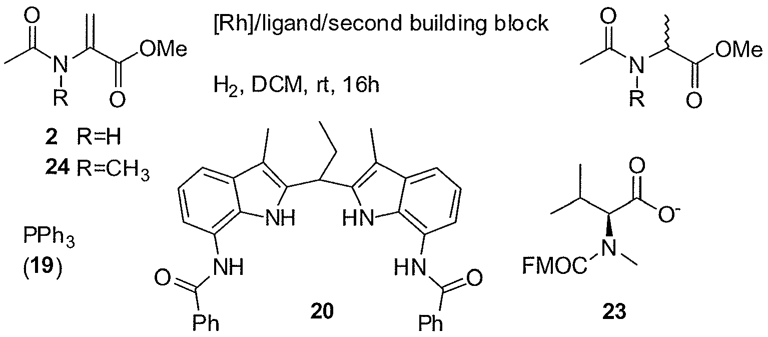 | | | | |

| substrate | first building block | second building block | % conv. | % ee (config) |
|---|---|---|---|---|
| 2 | 19 | 6 | 64 | 0 (-) |
| 2 | 19 | 18 | <1 | 0^{b} (-) |
| 2 | 19/20 | 6 | 11 | 0 (-) |
| 2 | 19/20 | 18 | <1 | 0^{b} (-) |
| 2 | - | 6 | 100 | 0 (-) |
| 2 | - | 18 | 4 | 0 (-) |
| 2 | 1 | 23 | 100 | 37 (S) |
| 24 | 1 | 6 | 100 | 0 (-) |
| 24 | 1 | 18 | 5 | <5 (R) |

| | | | | |
|---|---|---|---|---|
| ^{a} Reactions were performed in DCM, Rh/second building block/DIPEA/substrate = 1:1.1:12:9:100; first building block/Rh for 1 and for 19/20, 1.1 and 6/6, respectively; C(Rh)=0.001M, 10 bar H₂, at RT for 16 h, using [Rh(nbd)₂BF₄] as metal precursor; DIPEA was used as a base to deprotonate acidic second building block. Conversion and ee were determined by chiral GC analysis of the reaction mixture. ^{b} Higher conversion and no selectivity was observed when 18/DIPEA/Rh = 3:2:1 ratio was used. | | | | |

Further experiments were performed with other substrates. Instead of **2**, other substrates (S1-S6) were hydrogenated with high enantio-selectivity in the presence of different second building blocks.

**Table 3. Asymmetric hydrogenation of S1-S6 using [Rh(1)(nbd)]⁺ and first building block 1 (Fig. 2) in combination with various second building blocks^{a}.**

| | | | | | | | | | | | | |
|---|---|---|---|---|---|---|---|---|---|---|---|---|
| 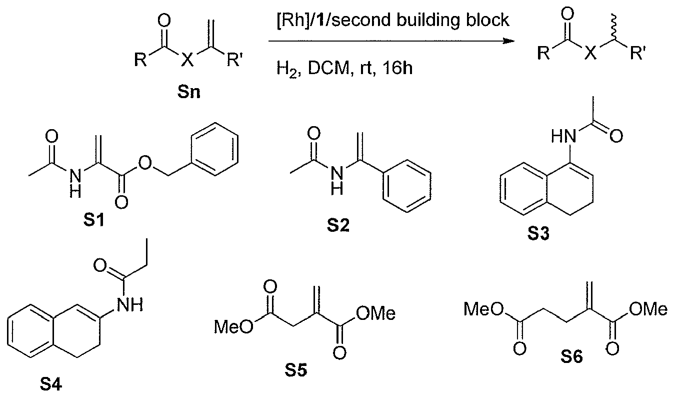 | | | | | | | | | | | | |

| Substrate | S1 | | S2 | | S3 | | S4 | | S5 | | S6 | |
|---|---|---|---|---|---|---|---|---|---|---|---|---|
| Second building block | conv. (%) | ee (%) | conv (%) | ee (%) | conv (%) | ee (%) | conv (%) | ee (%) | conv (%) | ee | conv (%) | ee (%) |
| 3 | 98 | 0(/) | 100 | 4(S) | | | 11 | 8 (+) | 100 | 0(/) | 100 | 0(/) |
| 4 | 98 | 0(/) | 97 | 0 (/) | | | 9 | 0(/) | 100 | 3(-) | 98 | 0(/) |
| 5 | 99 | 10 (R) | 100 | 6 (S) | | | 69 | 3 (+) | 90 | 0(/) | 100 | 0(/) |
| 6 | 100 | 47 (R) | 100 | 56 (S) | 96 | 78 (-) | 46 | 24 (+) | 98 | 17 (+) | 65 | 3 (+) |
| 7 | 99 | 39 (R) | 100 | 60 (S) | 100 | 62 (-) | 47 | 29 (+) | 98 | 14 (+) | 92 | 2 (+) |
| 8 | 99 | 35 (R) | 100 | 36 (S) | 100 | 63 (+) | 36 | 32 (+) | 88 | 7 (+) | 64 | 2 (+) |
| 9 | 99 | 0(/) | 100 | 0 (/) | 100 | 4(-) | 51 | 4 (+) | 100 | 0 (/) | 100 | 0(/) |
| 10 | 99 | 7(S) | 100 | 26 (R) | 100 | 40 (-) | 11 | 17 (+) | 100 | 0(/) | 100 | 4 (+) |
| 12 | 99 | 0(/) | 100 | 0(/) | 95 | 3(-) | 10 | <5(+) | 100 | 0(/) | 100 | 2 (+) |
| 13 | 3 | 0(/) | 100 | 0(/) | 64 | 0(/) | 6 | 0(/) | 100 | 0(/) | 100 | 0(/) |
| 14 | 100 | 0(/) | 100 | 3(S) | 6 | <5(-) | 4 | <5(+) | 100 | 0(/) | 100 | 0(/) |
| 15 | <2 | - | 100 | 33 (S) | 66 | 38 (-) | 7 | 15(+) | 100 | 7 (+) | 100 | 7(-) |
| 16 | 100 | 12 (S) | 6 | <5 (R) | 0 | - | 0 | - | 16 | 4 (+) | 5 | 0(/) |
| 17 | 100 | 5(R) | 7 | <5(R) | 0 | - | 0 | - | 32 | 0(/) | 18 | 0(/) |
| 18 | 43 | 90 (S) | 98 | 82 (S) | 0 | - | 7 | 22 (+) | 100 | 40(-) | 100 | 49 (-) |

| | | | | | | | | | | | | |
|---|---|---|---|---|---|---|---|---|---|---|---|---|
| ^{a} Reactions were performed in dichloromethane (DCM), Rh/1/second building block/ N,N-diisopropylethylamine (DIPEA)/Sn =1:1.1:3:2:100; C(Rh)=0.001M, 10 bar H₂, at RT for 16 h, using [Rh(nbd)₂BF₄] as metal precursor; DIPEA was used as a base to deprotonate acidic second building block. Conversion and ee were determined by chiral GC or chiral HPLC analysis of the reaction mixture; (-)=levorotary, (+)=dextrorotatory, (/)= optical inactive. | | | | | | | | | | | | |

The formation of a hydrogen bond between the NH of the substrate and the (thio)carbonyl functionality of the second building blocks may play a crucial role in the selectivity of the reaction. In many case the selectivity is further improved when the coordination complex also comprises the substrate to be chemically converted. In that case the substrate contains a functional group which forms a non-covalent bond with a complementary functional group of the second building block (which of course is another functional group that the one that already has formed a non-covalent bond to the first building block.

The best second building blocks usually have the strongest interaction with the complex, i.e. the best second building block should be able to dominate the catalysis in a competition experiment where a mixture of second building blocks is present in solution. It was found that even when a mixture of twelve different second building blocks was used (some of them giving opposite product isomers), the reaction outcome was strongly defined by the best second building block present producing the product with an ee of 81%).

Remarkably, the best second building block was chosen even though its close analogues were present in the reaction mixture. Such selection is of interest as it forms the basis for an iterative deconvolution screening strategy, as claimed in claim 11, and allowing identification of the optimal catalyst from a large library by performing only a limited number of experiments. In Figure 4 such method is schematically shown for a library of 12 second building blocks. When performing experiments in which the mixtures of second building blocks was divided each time in two batches, stepwise converging to the best second building block.

Following the best set of second building blocks gradually leads the ee from 81, 85, 88 to 98% ee in only 3 or 4 experiments, whereby the best second building block has been found in these few experiments.

## Claims

1. A coordination complex system for chemically converting a substrate comprising a non-protein ligand having a first building block with at least one functional group and at least one donor moiety, which donor moiety is complexed to a metal selected from a transition metal and lanthanide or an organometallic precursor thereof, **characterized in that** the ligand further comprises at least one second building block not having a donor moiety and having at least one functional group that is complementary to the at least one functional group of the first building block, wherein the first building block and the second building block are non-covalently bonded through their functional groups.

2. The coordination complex system of claim 1 wherein the second building block is chiral.

3. The coordination complex system of claim 1 wherein the first building block is chiral and the second building block is achiral.

4. The coordination complex system of any one of claims 1-3 comprising an organometallic precursor of the metal.

5. The coordination complex system of any one of claims 1-4 wherein the molar ratio between the first building block and the metal or the organometallic precursor thereof, is between 0.2 and 100.

6. The coordination complex system of any one of claims 1-5 further comprising the substrate to be chemically converted wherein the second building block contains at least a further functional group which is able to non-covalently bind the substrate.

7. Use of the coordination complex system of any one of claims 1-6 as a catalyst for hydroformylation, hydrogenation, transfer hydrogenation, hydrocyanation, polymerization, isomerization, carbonylation, cross-coupling, metathesis, CH-activation, allylic substitution, aldol condensation, or Michael addition of the substrate.

8. A method of preparing a catalyst by
a) selecting from a library a non-protein first building block having at least one functional group and at least one donor moiety,
b) selecting from a library a non-protein second building block not having a donor moiety and having at least one functional group that is complementary to the functional group of the first building block,
c) forming the catalyst by bringing in contact in any order the first building block, the second building block and a metal selected from a transition metal and lanthanide or an organometallic precursor thereof, by forming a non-covalent bond between a functional group of the first building block and a functional group of the second building block, which functional group is complementary to the functional group of the first building block, and by forming a complex between at least one of the donor moieties of the first building block and the metal or the organometallic precursor thereof.

9. A method of preparing a catalyst by
a) selecting from a library a non-protein first building block having at least one functional group and at least one donor moiety,
b) forming the catalyst by bringing in contact in any order the selected first building block, a plurality of second building blocks, each not having a donor moiety and having at least one functional group that is complementary to the functional group of the first building block, and a metal selected from a transition metal and lanthanide or an organometallic precursor thereof, by forming a non-covalent bond between a functional group of the first building block and a functional group of at least one of the second building blocks, which functional group is complementary to the functional group of the first building block, and by forming a complex between at least one of the donor moieties of the first building block and the metal or the organometallic precursor thereof.

10. A method of preparing a catalyst by
a) selecting from a library a second building block not having a donor moiety and having at least one functional group,
b) forming the catalyst by bringing in contact in any order the selected second building block, a plurality of first building blocks, each having at least one functional group that is complementary to a functional group of the second building block, and at least one donor moiety, and a metal selected from a transition metal and lanthanide or an organometallic precursor thereof, by forming a non-covalent bond between a functional group of the second building block and a functional group of at least one of the first building blocks, which functional group is complementary to the functional group of the second building block, and by forming a complex between at least one of the donor moieties of the first building block and the metal or the organometallic precursor thereof.

11. A method of selecting a second building block for a certain catalytic reaction with a certain substrate for use in the preparation of the coordination complex system of any one of claims 1-6, from a library of n second building blocks, wherein n is an integer of at least 3, by performing the following steps:
i) dividing the library of n second building blocks in a sub-library with m second building blocks and a sub-library with (n-m) second building blocks, wherein n and m are integers;
ii) using the libraries with m and (n-m) second building blocks in the method of claim 8 to prepare a catalyst library A and a catalyst library B, respectively;
iii) performing the certain catalytic reaction with the certain substrate using catalyst library A and the similar catalytic reaction with said substrate using catalyst library B, and selecting on the basis of the results of the catalytic reaction one out of catalyst library A or B;
iv) dividing the selected catalyst library A (or B when selected) with m (or (n-m)) second building blocks in a sub-library with p second building blocks and a sub-library with (m-p) (or (n-m-p)) second building blocks, wherein p is an integer;
v) preparing catalyst library C and catalyst library D from the sub-library with p second building blocks and the sub-library with (m-p) (or (n-m-p)) second building blocks, respectively, according to the method of claim 9, performing the catalytic reaction of step c) using catalyst library C and catalyst library D, and selecting on the basis of the results of the catalytic reaction one out of the catalyst library C or D;
vi) dividing the selected catalyst library C (or D when selected) with p (or (m-p) or (n-m-p)) second building blocks in a sub-library with q second building blocks to give library E and a sub-library with p-q (or (m-p-q) or (n-m-p-q)) second building blocks to give library F, wherein q is an integer, and repeat step v) with the sub-library with q second building blocks and the sub-library with (p-q) (or (m-p-q) or (n-m-p-q)) second building blocks to select one out of catalyst library E and catalyst library F;
vii) repeat step vi), and then steps v) and vi) until the selected catalyst library contains 1 second building block, which is the selected second building block for the certain catalytic reaction with the certain substrate.
